# EUROPEAN PATENT SPECIFICATION

(11) **EP 4 604 889 B1**
(45) Date of publication and mention of the grant of the patent: **15.07.2026**
(21) Application number: 23783422.1
(22) Date of filing: 04.10.2023
(51) Int. Cl.: A61F 13/00, A61F 13/02

(54) **A DRESSING COMPRISING A SKIN AND/OR WOUND BENEFICIAL COATING**
VERBAND MIT EINER HAUT- UND/ODER WUNDPFLEGEBESCHICHTUNG
PANSEMENT COMPRENANT UN REVÊTEMENT BÉNÉFIQUE POUR LA PEAU ET/OU LES PLAIES

(30) Priority: 17.10.2022 EP 22201868
(43) Date of publication of application: 27.08.2025
(73) Proprietor: Mölnlycke Health Care AB, 431 21 Mölndal (SE)
(72) Inventor: SÖDERSTRÖM, Bengt, 435 41 MÖLNLYCKE (SE); JOHANNISON, Ulf, 438 91 LANDVETTER (SE); JÖRGENSEN, Steen, 418 71 GÖTEBORG (SE)
(74) Representative: Kransell & Wennborg KB
(86) International application number: PCT/EP2023/077427
(87) International publication number: WO 2024/083506

(56) References cited:
- EP-B1- 2 489 339
- WO-A1-2008/148797
- WO-A1-94/09848

## Description

### TECHNICAL FIELD

The present disclosure generally relates to a medical dressing comprising a top layer, an adhesive skin contact layer, and a release liner detachably attached to at least a portion of the adhesive skin contact layer; the adhesive skin contact layer having a first side facing the top layer and a second side facing the release liner, wherein the second side of the adhesive skin contact layer is provided with a coating comprising a skin and/or wound beneficial agent configured to be released from the adhesive skin contact layer during use. The present disclosure also relates to a process for manufacturing a medical dressing.

### BACKGROUND

Adhesive medical dressings are frequently used in wound care, both for the purpose of treating wounds and scars and for the purpose of preventing wounds from occurring in the first place.

Various types of dressings exist on the market. Some dressings, commonly referred to as "film dressings", typically comprise a backing layer (top layer) and an adhesive skin contact layer. Absorbent dressings typically comprise an absorbent pad arranged between the backing layer and the adhesive skin contact layer. In a so called "bordered dressings", the backing layer and the adhesive skin contact layer are configured to extend beyond the contour of the absorbent pad.

The purpose of the adhesive skin contact layer is to adhere to the skin or the wound of a wearer and to fixate the dressing in a desirable position.

In the treatment or prevention of a wound, various skin and/or wound beneficial agents may be utilized and incorporated in the dressing.

For example, in chronic and surgical wounds, infection is a common problem. A surgical site or an open wound is a suitable environment for bacteria to accommodate and colonize. A bacterial infection in the wound or the skin surrounding the wound may disrupt the normal wound healing process and result in chronic, non-healing wounds.

To prevent wound infection, antimicrobial agents are often used to eliminate or reduce infection of the wound. The incorporation of antimicrobial agents into a medical dressing may promote healing and eliminate or reduce the risk of infection of the wound. EP2489339 and WO2008148797 disclose examples of such dressings.

In order to prevent a wound from occurring in the first place, various types of skin beneficial agents may be utilized.

Skin and/or wound beneficial agents may be incorporated into a layer in the medical dressing, e.g. a wound pad layer. Alternatively, such agents may be applied as a coating on the adhesive layer of the medical dressing; i.e. the layer arranged in contact with the skin or wound during use.

**In** many instances, a quick delivery of the skin and/or wound beneficial agent is desirable. **In** this regard, the provision of a coating on the adhesive layer may be the desired mode of integration of the agent in the medical dressing.

During assembly of the medical dressing, the adhesive layer is typically covered by a release liner to protect the adhesive layer and the dressing from contamination prior to use. Prior to use, the medical dressing is sterilized.

When applying a coating comprising a skin and/or wound beneficial agent to the adhesive layer of a medical dressing, it is important that the coating does not impair the adhesion properties or the "tackiness" of the adhesive layer.

Furthermore, it is important that the coating comprising the skin and/or wound beneficial agent remains on the adhesive surface of the medical dressing when the dressing is to be applied to the skin or wound of a patient.

One problem associated with medical dressings comprising coatings on the adhesive surface is that the coating is transferred from the adhesive surface to the release liner. Accordingly, when the release liner is removed from the dressing, the skin and/or wound beneficial agent present in such a coating is removed with the release liner, and thereby removed from the adhesive layer. As a consequence, the skin and/or wound beneficial effect is lost or is at least significantly reduced.

It is therefore a need to overcome the above-mentioned challenges with medical dressings comprising skin and/or wound beneficial coatings on the adhesive surface. More particularly, there is a need to provide a medical dressing that provides a quick delivery of a skin and/or wound beneficial agent, wherein the effect remains in the medical dressing even after disassembly of the dressing; i.e. even after the removal of the release liner.

### SUMMARY

The invention is defined by the appended claims.

**In** view of the above mentioned problems, it is an object of the present disclosure to provide improvements with respect to medical dressings comprising skin and/or wound beneficial agents and securing that the effect of such skin and/or wound beneficial agents is maintained in the dressing when the dressing is applied to the skin or wound of a patient.

According to a first aspect, there is provided a medical dressing comprising a top layer, an adhesive skin contact layer and a release liner detachably attached to at least a portion of the adhesive skin contact layer; the adhesive skin contact layer having a first side facing the top layer and a second side facing the release liner, wherein the second side of the adhesive skin contact layer is provided with a coating comprising a skin and/or wound beneficial agent configured to be released from the adhesive skin contact layer during use, wherein the release liner has a first side facing the second side of the adhesive skin contact layer, and an opposing, second side, wherein the first side of the release liner comprises a plurality of protrusions.

The present invention is based on the realization that a release liner comprising a plurality of protrusions on the side facing the skin-facing (second) side of the adhesive skin contact layer significantly improves the ability of the coating to remain on the surface of the adhesive skin contact layer even after the removal of the release liner.

With a conventional dressing, the release liner is typically flat and fully adherent to the adhesive dressing surface. When such a release liner is to be removed from the adhesive surface, a large removal force is generally required, and the skin and/or wound beneficial coating has an enhanced tendency to migrate to the release liner during removal thereof.

The medical dressing of the present disclosure yields a reduced attachment between the release liner and the adhesive skin contact layer, and the skin and/or wound beneficial coating is largely prevented from becoming transferred to the release liner. The release liner is still sufficiently adherent and attached to the adhesive skin contact layer in order to prevent the entry of contaminants into the dressing.

**In** exemplary embodiments, the coating may be a discontinuous coating.

A discontinuous coating is beneficial to secure that the adhesive skin contact layer maintains its adhesion properties, and that the tackiness of the adhesive layer is maintained despite the presence of the coating.

**In** exemplary embodiments, the coating may be soluble in an aqueous medium.

Accordingly, in contact with wound exudate or moist skin, the coating dissolves and allows for a quick initial release of the skin and/or wound beneficial agent.

**In** exemplary embodiments, the skin and/or wound beneficial agent is an antimicrobial agent, preferably an antimicrobial salt.

Antimicrobial agents, such as antimicrobial salts, are beneficial to prevent wound infection. When the antimicrobial agent is released from adhesive skin contact layer, bacteria present at the wound or at the skin surrounding the wound can be eradicated. Growth of infecting microorganisms at the surface of the adhesive skin contact layer is thereby prevented, which avoids colonization at the wound site and within the wound medical dressing.

In exemplary embodiments, the concentration of the skin and/or wound beneficial agent in the coating may be from 1 to 1000 µg/cm2, preferably from 5 to 500 µg/cm2, more preferably from 10 to 200 µg/cm2.

Accordingly, a sufficient release of the skin and/or wound beneficial agent is enabled when the coating dissolves in contact with wound fluid or skin moisture. Furthermore, this range provides for a good balance between a sufficient release of the agent, as well as maintaining the adhesive properties of the adhesive skin contact layer.

The plurality of protrusions are spaced apart from one another by non-protruding areas, wherein the non-protruding areas form gaps between the first side of the release liner and the second side of the adhesive skin contact layer.

Accordingly, the protrusions are detachably attached to the adhesive skin contact layer and the non-protruding areas are unattached to the adhesive skin contact layer.

The gaps defined between the second side of the adhesive skin contact layer and the first side of the release liner reduces the contact area between the release liner and the adhesive skin contact layer. By reducing the adherence between these layers, the inventors have found that the skin and/or wound beneficial agent has a significantly enhanced ability to stay; i.e. remain coated on the adhesive skin contact layer instead of migrating to the release liner (and thereby removed from the dressing). Accordingly, the wound and/or skin beneficial effect is maintained in the dressing.

In exemplary embodiments, the first side of the release liner is detachably attached to from 5 to 75 %, preferably from 10 to 70%, most preferably from 10 to 50% of the surface area of the second side of the adhesive skin contact layer.

Accordingly, the release liner serves the dual function of protecting the adhesive skin contact layer, while also preventing the skin and or wound beneficial coating from migrating to the release liner.

In exemplary embodiments, the height of each protrusion may be from 0.1 to 2.0 mm, preferably from 0.2 to 1.5 mm, most preferably from 0.3 to 1.2 mm.

If the protrusions are too high, they may create indentations and imprints in the adhesive skin contact layer, which is undesirable when the dressing is attached to the skin. Such imprints may reduce the adherence to the skin of a patient, and the ability of the adhesive skin contact layer to conform to the skin may be impaired.

**In** contrast, if the height is too small, the gaps defined by the non-protruding areas of the first side of the release liner may be reduced, and the attachment of the first side of the release liner to the adhesive skin contact layer may increase.

Furthermore, a protrusion height in the above-mentioned range is suitable to ensure that the release liner prevents the entry of contaminants into the dressing.

**In** exemplary embodiments, the medical dressing may comprise an absorbent pad arranged between the top layer and the adhesive skin contact layer, and wherein the plurality of protrusions are arranged in an area on the first side of the release liner that underlies the absorbent pad.

The absorbent pad serves the purpose of dealing with large amounts of wound liquid exuded by the wound. Since many wounds, particularly infected wounds, may exude large amounts of exudate, it is typically necessary to utilize a dressing comprising an absorbent pad. The absorbent pad may comprise any absorbent material, such as absorbent fibers, gels, foams etc. The absorbent pad may comprise one or more pad-forming layers.

Where the dressing comprises an absorbent pad, the skin and/or wound beneficial effect is suitably arranged in an area of the adhesive skin contact layer that underlies the absorbent pad. **In** this regard, the plurality of protrusions is preferably arranged to cover this area, which is often the central portion of the dressing.

**In** exemplary embodiments, the adhesive skin contact layer comprises a silicone-based adhesive coating.

A silicone-based adhesive coating is soft and gentle to the skin and can be removed without causing trauma.

However, the softness of the silicone-based adhesive may render the adhesive skin contact layer more prone to adapt to the contour of an underlying surface structure. This characteristic is beneficial in the application against the skin or wound of a patient, particularly a curved or contoured skin surface. However, it is undesired if the adhesive skin contact layer conforms "too much" to the tops and valleys defined by the protrusions on the first side of the adhesive skin contact layer, since this may result in a too firm adhesion between the release liner and the adhesive skin contact layer. This may result in undesired migration; i.e. transfer of the coating to the adhesive skin contact layer.

**In** exemplary embodiments, the adhesive skin contact layer may comprise a polymeric film provided with a silicone-based adhesive coating.

The polymeric film yields stability to the applied silicone-based adhesive coating, and counter-acts the tendency of the silicone adhesive to follow the contour of the protrusions. Accordingly, the polymeric film prevents the silicone-based adhesive from sinking deeper into the valleys defined by the non-protruding parts of the first side of the release liner. The inventors have found that this feature is important to prevent the migration of skin and wound beneficial agents applied as coatings to the release liner.

In exemplary embodiments, the silicone-based adhesive coating has a thickness of from 35 to gsm to 300 gsm, preferably from 45 to 250 gsm.

If the thickness of the silicone-based adhesive coating is too low, poor adhesion to the skin may result. In contrast, a thicker silicone-based adhesive coating has an increased tendency to follow the contour of the protrusions. Furthermore, the risk of the protrusions forming imprints in the silicone-based adhesive skin contact layer is enhanced with a thicker silicone-based coating.

In exemplary embodiments, each of the plurality of protrusions has a substantially flat top surface; the top surface forming the contact surface for detachable attachment of the release liner to the adhesive skin contact layer.

This is particularly advantageous in embodiments where a thicker silicone-based adhesive coating is utilized. Since a thicker silicone-based adhesive coating has an enhanced tendency to "sink into" the gaps defined by the non-protruding areas of the first side of the release liner, this behavior is counteracted by protrusions having a substantially flat top surface.

According to another aspect, there is provided a process for manufacturing a medical dressing comprising:
a) providing a dressing comprising a top layer and an adhesive skin contact layer; the adhesive skin contact layer having a first side facing the top layer and a second, opposing side,
b) applying a coating onto the second side of the adhesive skin contact layer, wherein the coating comprises a skin and/or wound beneficial agent configured to be released from the adhesive skin contact layer during use,
c) providing a release liner having a first side and an opposing second side, wherein at least a portion of the first side of the release liner comprises a plurality of protrusions,
d) applying the release liner onto the adhesive skin contact layer such that the plurality of protrusions become detachably attached to the second side of the adhesive skin contact layer.

In exemplary embodiments, step b) of applying a coating is performed by spray coating.

This coating technique is beneficial as it allows for flexibility depending on the dressing to be used and depending on the type of wound to be treated. It is also a simple means to apply the coating. Selected areas of the adhesive skin contact layer may be coated and the size of the droplets on the surface may be controlled to avoid interfering with the adhesive properties of the adhesive skin contact layer
In exemplary embodiments, the protrusions are formed as an in-line step in the process, preferably by means of embossing.

The provision of protrusions in line has several advantages from a process and storage perspective. If the protrusions are provided in the release liner as a separate process step prior to the process of manufacturing the dressing, the protruded release liner would require much more space during storage; e.g. when stored on a storage roll. In contrast, if the plurality of protrusions are formed as an in-line step, the release liner may be stored on storage rolls in a flat condition.

Furthermore, embossing is a simple technique to form three dimensional features, e.g. protrusions in a layer, and is suitable for use where the protrusions are formed as an in-line step in the process.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 conceptually illustrates the problem associated with dressings of the prior art.
Figure 2 illustrates a split-view of a medical dressing according to an exemplary embodiment of the present disclosure, seen from bottom-to-top.
Figure 3 illustrates a split-view of a medical dressing according to an exemplary embodiment of the present disclosure, seen from top-to-bottom, wherein the dressing further comprises an absorbent pad.
Figure 4a illustrates a medical dressing according to an exemplary embodiment of the present disclosure, as seen from the bottom side when the release liner is to be peeled off by a caregiver.
Figure 4b is a zoomed-in view of a portion of the dressing in figure 4a.
Figure 4c is a is a cross-sectional view of a portion of the medical dressing in figure 4a, showing the release liner being peeled off from the adhesive skin contact layer.
Figure 5 illustrates a scanning electron microscope (SEM) image of a release liner having a pyramidal shaped protrusion pattern, used for Examples 1 and 2.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

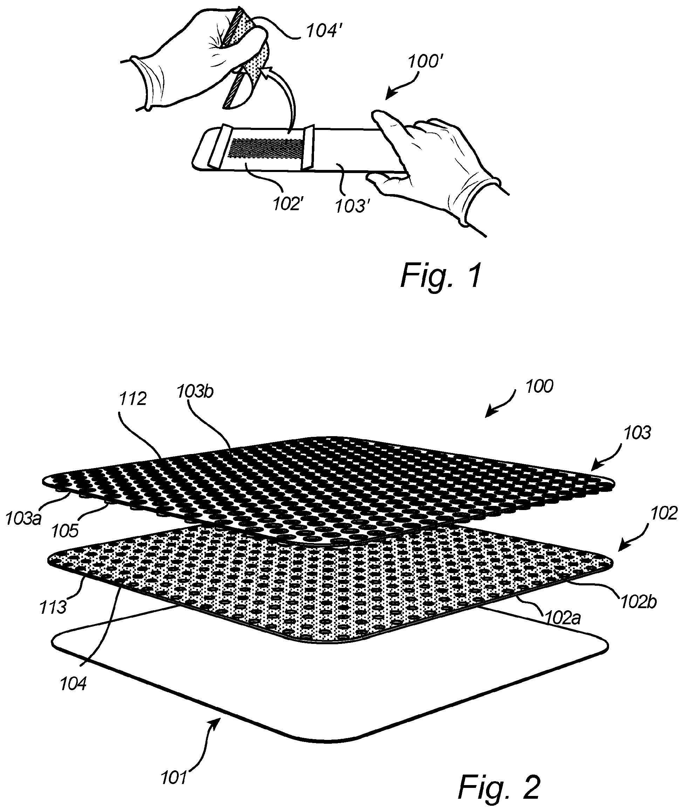

The problem underlying the present disclosure is schematically illustrated in figure 1. Figure 1 illustrates a dressing 100' according to the prior art comprising an adhesive skin contact layer 102' and a release liner 103'. The release liner illustrated in figure 1 comprises three removable portions, and is attached to the adhesive skin contact layer 102' of the dressing. The adhesive skin contact layer 102' initially comprises a coating comprising a skin and/or wound beneficial agent on the skin-facing surface. When a first release liner portion is removed, the coating comprising the beneficial agent is removed from the adhesive skin contact layer 102' (illustrated by the arrow in figure 1) and transferred to the surface of the removed release liner portion (see 104'). Consequently, the skin and/or wound benefical effect of the dressing is eliminated.

Figure 2 illustrates a medical dressing according to an exemplary embodiment of the present disclosure. The medical dressing 100 comprises a top layer 101, an adhesive skin contact layer 102 and a release liner 103 detachably attached to at least a portion of the adhesive skin contact layer 102; the adhesive skin contact layer having a first side 102a facing the top layer and a second side 102b facing the release liner 103, wherein the second side 102b of the adhesive skin contact layer 102 is provided with a coating 104 comprising a skin and/or wound beneficial agent configured to be released from the adhesive skin contact layer 102 during use, wherein the release liner 103 has a first side 103a facing the second side 102b of the adhesive skin contact layer 102, and an opposing, second side 103b, wherein the first side 103a of the release liner comprises a plurality of protrusions 105.

As used herein, the term "top layer" means the uppermost layer of the medical dressing; i.e. the layer facing away from the patient's skin during use. The top layer may also be referred to as a backing layer.

The adhesive skin contact layer is the layer that is to be arranged in contact with the patient's skin. The skin contact layer may also be referred to as a wound contact layer.

The top layer and the adhesive skin contact layer are typically co-extensive. The surface area of the top layer is the same as the surface area of the adhesive skin contact layer.

The release liner is arranged to cover the entire surface area of the adhesive skin contact layer.

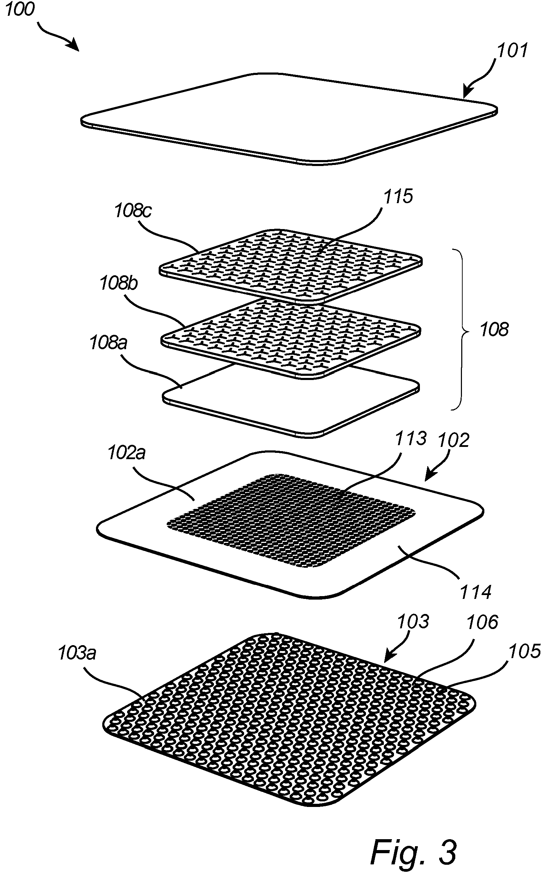

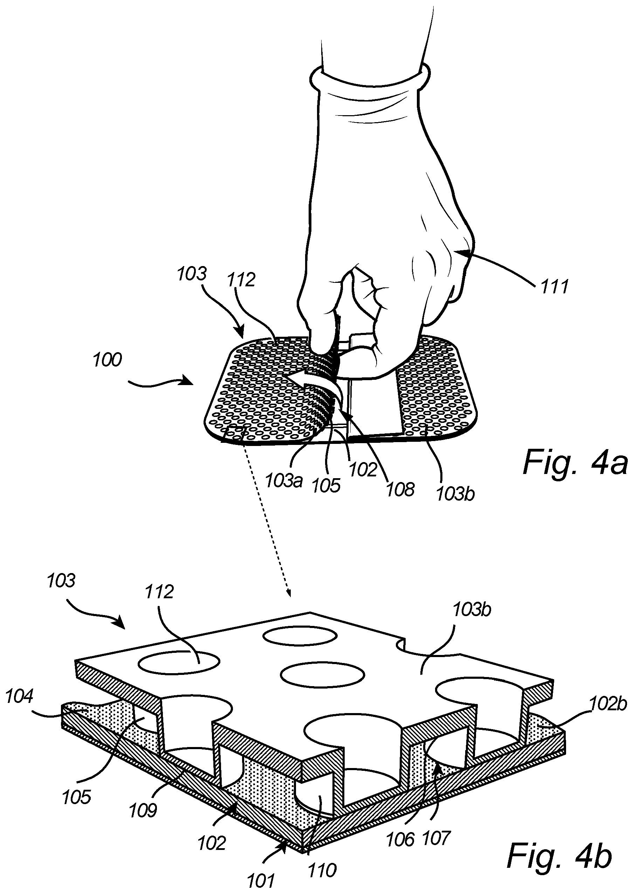

The release liner may comprise one or a plurality of removable portions. In figures 2 and 3, the release liner contains one removable portion, whereas in figure 4a, the release liner contains two removable portions.

If the first release liner comprises more than one removable portion, the first release liner may be divided by a dividing line, wherein a first and a second removable portion of the release liner overlaps along the dividing line. The overlapping removable portions secure that the adhesive skin contact layer is fully covered and that no gaps are formed, thereby preventing contaminants from entering the dressing. As illustrated in figure 4a, the overlapping removable portions may form a grip member that the applicator, e.g. caregiver 111 can grasp in order to remove and separate the removable portion. For example, an edge of the second removable portion may be folded over itself, and the edge of the first removable portion may be configured to overlap the folded edge.

Regardless of how many removable portions the release liner contains, the release liner is arranged to cover the entire adhesive skin contact layer.

The dressing of the present disclosure may be used on all types of wounds, incisions and even on intact skin (if the purpose of the dressing is to prevent wounds from occurring in the first place).

As used herein, the term "skin and/or wound beneficial agent" means any agent that has a skin and/or wound beneficial effect. The skin and/or wound beneficial agent may prevent a wound from forming in the first place, improve the wound healing process or treat an existing wound.

For example, the skin and/or wound beneficial agent may be an antimicrobial, pH buffering, immunomodulating, anti-inflammatory agent, a vitamin, a growth factor, an agent affecting angiogenesis, migration of cells etc.

The coating 104 may be a discontinuous coating.

As used herein, the term "discontinuous coating" means that the coating does not fully cover the skin-facing surface of the adhesive skin contact layer.

A discontinuous coating is generally preferred to avoid impairing the adhesion to the skin afforded by the adhesive skin contact layer.

The coating 104 may be soluble in an aqueous medium.

As used herein, the term "soluble in an aqueous medium" means that the coating dissolves in contact with an aqueous medium. The aqueous solution may be water. Accordingly, the coating dissolves quickly in contact with wound exudate or skin moisture. Even low amounts of skin moisture or wound exudate will induce dissolution of the coating. The coating comprising the skin and/or wound beneficial agent may initially be applied as an aqueous solution to the surface of the adhesive skin contact layer, and subsequently dried. In contact with wound exudate or an aqueous medium, the dried coating will be dissolved and the skin and/or wound beneficial agent will be released from the adhesive skin contact layer.

Accordingly, in contact with wound exudate or moist skin, the coating dissolves and allows for a quick initial release of the skin and/or wound beneficial agent.

For example, at least 60%, e.g. at least 80%, e.g. at least 100% of the coating 104 may be configured to be dissolved within 3 hours of exposure to an aqueous medium.

In order to prevent or alleviate wound infection, the skin and/or wound beneficial agent may be an antimicrobial agent, preferably an antimicrobial salt.

The antimicrobial agent may be any agent with the ability to prevent or slow down the growth of bacteria, or kill off bacteria present at the wound site.

Examples of antimicrobial salts include silver salts, PHMB salts, chlorhexidine salts etc.

In exemplary embodiments, the antimicrobial salt is a chlorhexidine salt.

The present disclosure may be particularly suitable for chlorhexidine salts, e.g. chlorhexidine gluconate, which has an enhanced tendency of migrating from the adhesive surface to the release liner.

The concentration of the skin and/or wound beneficial agent in the coating 104 may be from 1 to 1000 µg/cm2, e.g. from 5 to 500 µg/cm2, e.g. from 10 to 200 µg/cm2.

This secures the release of the skin and/or wound beneficial agent in an amount sufficient to yield a wound healing or wound beneficial effect at the wound site.

The concentration may vary depending on the skin and/or wound beneficial agent utilized. For certain agents, a low concentration is sufficient, whereas other agents may require a higher concentrations.

For example, if the skin and/or wound beneficial agent is an antimicrobial agent, the concentration in the coating may be in the range of from 30 to 200 µg/cm2.

As best illustrated in figure 3, the plurality of protrusions 105 are spaced apart from one another by non-protruding areas 106. The non-protruding areas form gaps between the first side 103a of the release liner 103 and the second side 102b of the adhesive skin contact layer 102.

The protrusions 105 are detachably attached to the second side 102b of the adhesive skin contact layer 102, and wherein the non-protruding areas 106 are unattached to the second side of the adhesive skin contact layer 102b.

Besides the advantages associated with a reduced migration of a skin and/or wound beneficial coating from the adhesive surface to the release liner, the inventors have also found that the provision of the protrusions on the second side of the release liner significantly facilitates the handling and application procedure of the dressing for a caregiver.

Adhesive medical dressings are typically thin and flimsy, and very difficult to handle since they are extremely prone to wrinkle formation. In contrast to conventional plasters or band-aids, where the release liner is fully removed prior to application of the dressing, medical dressings used in a care facility and/or a hospital setting, are applied in a different manner. The removal of the release liner is typically performed in a gradual or stepwise manner while simultaneously applying the dressing onto the skin or wound of a patient. The application and handling of the dressing is performed by a caregiver.

The medical dressing of the present disclosure yields a reduced attachment between the release liner and the adhesive skin contact layer. The delamination force; i.e. the force required to remove the release liner, is thus significantly reduced. The reduced delamination force prevents wrinkles from forming when the release liner is peeled off and the dressing is applied onto the skin of a patient.

As illustrated in figure 2 and in figures 4a-c, the second side 103b of the release liner 103 may comprise depressions 112. The depressions 112 of the second side 103b of the release liner 103 coincide with the protrusions 105 on the first side 103a of the release liner 103. The depressions 112 on the second side 103b may be formed when the release liner is embossed. For example, the release liner may be subject to an embossing tool on the second side of the release liner, which form protrusions 105 on the first side 103a and corresponding depressions 112 on the second side 103b of the release liner. The present disclosure is, however, not limited to embossed protrusions. In exemplary embodiments, the second side 103b of the release liner is flat.

The first side 103a of the release liner 103 may be detachably attached to from 5 to 75 %, preferably from 10 to 70%, most preferably from 10 to 50% of the surface area of the second side 102b of the adhesive skin contact layer 102.

Accordingly, the release liner 103 serves the dual function of protecting the adhesive skin contact layer from contamination, yet reducing the migration of skin and/or wound beneficial agents to the release liner. Furthermore, the removal of the release liner and application onto the skin of a patient is facilitated and wrinkles are prevented from forming.

The attachment between the first side of the release liner and the second side of the release liner may vary depending on the type of adhesive skin contact layer used, the thickness of the adhesive skin contact layer etc.

The height of each of the protrusions 105 may be from 0.1 to 2.0 mm, preferably from 0.2 to 1.5 mm, most preferably from 0.3 to 1.2 mm.

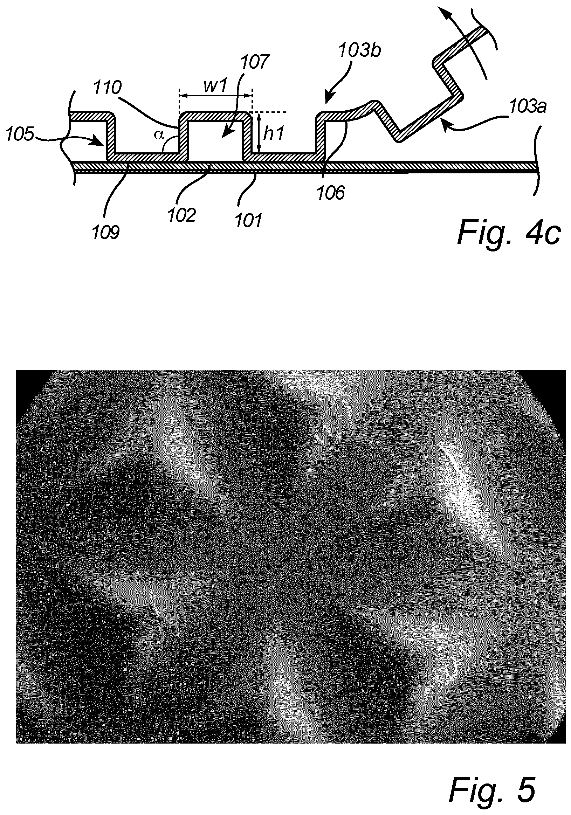

The height, h1, of one protrusion is illustrated in figure 4c.

The height means the maximum longitudinal extension of the protrusion. The height is measured from a top surface of the protrusion to the bottom portion of the protrusion. Accordingly, the height, h1, may be measured from the top surface 109 of the protrusion to the non-protruding area 106.

These dimensions are suitable for achieving well defined gaps between the protrusions (see 107 in figures 4b-c). If the height of the protrusion is too large, the risk of forming imprints in the adhesive skin-contact layer increases. In contrast, if the height is too small, the adhesive skin contact layer may become attached to the entire first side of the release liner, which is undesirable.

As best illustrated in figure 3, the medical dressing 100 may comprise an absorbent pad 108 arranged between the top layer 101 and the adhesive skin contact layer 102.

The dressing in figure 3 is a so called "border dressing". The top layer 101 and the adhesive skin contact layer 102 are configured to extend beyond the contour of the absorbent pad 108 to form a border portion.

In figure 3, the border portion of the adhesive skin contact layer 102 is denoted 114.

The absorbent pad 108 may be formed from a single layer or a plurality of pad-forming layers. The absorbent pad is not limited to a particular material, but typically comprises an absorbent foam or a gel. Alternatively, or additionally, the absorbent pad may comprise a superabsorbent material e.g. superabsorbent polymers (SAP) or superabsorbent fibers (SAF).

In exemplary embodiments, the absorbent pad comprises two or more layers having different properties.

As illustrated in figure 3, the absorbent pad 108 may comprise a first absorbent layer 108a, a liquid distributing layer 108b and a second absorbent layer 108c. The liquid distributing layer 108b may be arranged between the first 108a and the second 108c absorbent layer.

The first absorbent layer 108a may comprise a foam. Suitable foam materials for use in the first absorbent layer 108a include, but are not limited to polyurethane foams.

The second absorbent layer 108c may be a superabsorbent layer. Accordingly, the second absorbent layer may comprise superabsorbent polymers (SAP) or superabsorbent fibres (SAF).

The liquid distributing layer 108b may comprise any material having the ability to distribute the exudate in an efficient manner. For example, the liquid distributing layer 108b may comprise a nonwoven material. A nonwoven imparts an appropriately balanced rigidity to the layer and to the dressing as such. It may also efficiently distribute and spread liquid absorbed by the absorbent layer 108a such that it can be evaporated through the top layer 101 over a large surface. For example, the nonwoven may comprise viscose, polyester or blends thereof.

The layers can be joined by adhesion, lamination, using e.g. pressure and heat.

The absorbent pad may comprise additional layers, such as liquid transport layers, various combinations of foam and nonwoven layers laminated together.

With reference to figure 3, the layer 108a may comprise an absorbent foam, the layer 108b may be a liquid acquisition layer, and the layer 108c may be a superabsorbent layer.

A multi-layered pad prevents accumulation of body liquids close to the skin and improves the liquid handling of the dressing. Most wounds will contain some exudate, but the level of exudate may vary. In a chronic wound, the exudate production may be very large due to an ongoing inflammation. A dressing having the construction as explained above is suitable for handling large amounts of exudate and for preventing maceration of the skin surrounding the wound. Thus, the dressing is particularly suited for infection prevention.

As illustrated in figure 3, the absorbent pad 108 may comprise a plurality of cuts 115 extending at least partially through the absorbent pad 108. The cuts 115, which may have various shapes, render the pad more flexible, and enhances the flexibility of the entire dressing.

The cuts 115 illustrated in figure 3 each comprises three incisions extending from a common starting point. The angle between such incisions may be between 40 and 150°. Accordingly, the pad or pad layer(s) is/are cut in both the longitudinal (y) and lateral (x) directions of the pad such that the pad becomes flexible in all directions.

If the absorbent pad 108 comprises several pad-forming layers, the cuts 115 may extend through at least one of such layers.

Preferably, the plurality of protrusions 105 are arranged in an area on the first side 103a of the release liner 103 that underlies the absorbent pad 108.

Since the absorbent pad 108 overlies the wound site and is arranged to deal with the liquid exuded by the wound, the best wound healing effect is typically achieved if the coating and the protrusions are arranged in an area underlying the absorbent pad 108.

For the purpose of facilitating handling of the dressing and application onto the skin, the plurality of protrusions may be arranged in an area underlying the border portion of the dressing.

The plurality of protrusions 105 may be arranged in a pattern extending across the first side 103a of the release liner 103. The plurality of protrusions 105 may be arranged in a pattern extending across the entire first side 103a of the release liner 103, as illustrated in figure 3.

Typically, at least 50% of the first side 103a of the release liner 103 may comprise a pattern of protrusions 105.

The adhesive skin contact layer 102 may comprise a silicone-based adhesive coating.

Such an adhesive is skin-friendly and permits the removal of the dressing without causing damage to the skin. The softness of the silicone-based adhesive may be associated with some challenges since a soft silicone-based adhesive has an enhanced tendency to conform to an underlying surface, thereby making the coating 104 applied onto the silicone-based adhesive more prone to migrate to the release liner.

In the context of the present disclosure, such undesired migration is avoided by means of the gaps defined by the protrusions on the first side of the release liner.

The silicone-based adhesive coating may have a thickness of from 35 to 300 gsm, e.g. from 45 to 250 gsm.

Accordingly, an appropriate balance between skin adhesion, prevention of imprints in the silicone-based adhesive, and preventing the silicone-based adhesive from following the contour of the protrusions, is achieved.

The adhesive skin contact layer 102 may comprise a polymeric film provided with a silicone-based adhesive coating (not shown).

The polymeric film yields stability to the applied silicone-based adhesive coating, and prevents the silicone-based adhesive from following the contour of the protrusions. Accordingly, the polymeric film secures that the coating remains attached to the silicone-based adhesive coating when the release liner is removed from the dressing.

The polymeric film is preferably a breathable film and may comprise e.g. polyethylene, polyamide or polyurethane. Preferably, the polymeric film comprises polyurethane. The thickness of the polymeric film may be from 15 to 100 µm, e.g. from 20 to 80 µm, preferably from 20 to 50 µm.

The silicone-based adhesive coating of the adhesive skin contact layer is arranged to contact the skin of a wearer during use.

The protrusions on the first side of the release liner are not limited to a specific shape. However, in embodiments where a thicker silicone-based adhesive coating is utilized, it may be advantageous if the protrusions have a substantially flat top surface.

As best illustrated in figures 4a-4c, the protrusions have a substantially flat top surface 109.

Each of the plurality of protrusions 105 may have a top surface 109 and sidewalls 110 extending between the top surface 109 and the non-protruding area 106. The top surface 109 forms the contact surface for detachable attachment to the second side 102b of the adhesive skin contact layer 102.

As mentioned hereinbefore, if a thick silicone-based adhesive is utilized, e.g. a thickness above 200 µm, a flat protrusion top surface 109 is considered to be beneficial. First, because imprints in the silicone-based adhesive coating caused by too sharp or too angled protrusion top surfaces are avoided. Furthermore, the flat top surface secures that the adhesive skin contact layer lies flat on top of the plurality of protrusions without sinking into the valleys defined by the non-protruding areas. In this regard, the undesired transfer of the coating 104 applied onto the silicone-based adhesive of the adhesive skin contact layer 102 is significantly reduced.

As best illustrated in figure 4c, the angle, α, between the top surface 109 and the sidewalls 110 may be from 45 to 125 degrees, e.g. from 60 to 120 degrees, e.g. from 80 to 100 degrees.

If the angle, α, is too large, which may e.g. be the case with a patterned release liner having a "wavy" pattern, the adhesive skin contact layer has a tendency to conform to the contour of the wavy pattern. In this regard, the delamination force between the adhesive skin contact layer and the release liner may actually increase and a larger surface area of the second side of the adhesive skin contact layer may become attached to the first side of the release liner.

The width, w1, of the protrusion may be in the range of from 0.7 to 3.0 mm.

The width, w1, is the maximum lateral extension of one protrusion 105.

The surface area of the top surface 109 of each protrusion 105 may be from 0.1 to 2.5 mm2, e.g. from 2.0 to 2.0 mm2, e.g. from 0.3 to 1.6 mm2.

A surface area in the above-mentioned range improves the detachable attachment to the adhesive skin contact layer 102. If the surface area is too large, the first side of the release liner may become too firmly attached to the second side of the adhesive skin contact layer. As a result, the risk of the coating becoming transferred to the release liner increases.

If a thicker silicone-based adhesive is utilized, the surface area of the top surface 109 may be in the higher range (to prevent the adhesive from sinking into the gaps defined by the non-protruding area). In contrast, if a thinner silicone based adhesive (thickness below 200 µm, e.g. below 150 µm), the surface area of the top surface 109 may be in the lower range (to prevent coating transfer).

The top surface 109 of each protrusion is not limited to a particular shape, but any shape is conceivable, e.g. circular, oval, square, rectangular etc .

The distance between one protrusion and an adjacent protrusion may be from 1.0 to 8.0 mm, e.g. from 1.0 to 3.0 mm. The distance between adjacent protrusions depends on the dimensions of the protrusions, the size of the dressing etc. The distance between one protrusion and an adjacent protrusion may correspond to the diameter or the maximal width of the protrusion.

In exemplary embodiments, the plurality of protrusions may be formed by embossing.

Embossing is a simple and reliable technique enabling the provision of distinct, three dimensional protrusions in the release liner that remain in place during e.g. storage and transport of the dressing.

According to another aspect, there is provided a process for manufacturing a medical dressing 100 comprising:
a) providing a dressing 100 comprising a top layer 101 and an adhesive skin contact layer 102; the adhesive skin contact layer having a first side 102a facing the top layer and a second, opposing side 102b,
b) applying a coating 104 onto the second side 102b of the adhesive skin contact layer 102, wherein the coating 104 comprises a skin and/or wound beneficial agent configured to be released from the adhesive skin contact layer 102 during use,
c) providing a release liner 103 having a first side 103a and an opposing second side 103b, wherein at least a portion of the first side 103a of the release liner 103 comprises a plurality of protrusions 105;
d) applying the release liner 103 onto the adhesive skin contact layer 102 such that the plurality of protrusions 105 become detachably attached to the second side 102b of the adhesive skin contact layer 102.

The dressing may be provided by means known to the skilled person (step a). The dressing may also comprise an absorbent pad between the top layer and the adhesive skin contact layer. The assembly of the top layer, the adhesive skin contact layer, and the absorbent pad, where present, is not limited to a particular method, but any means (e.g. adhesive bonding, lamination etc.) may be utilized.

The skin and/or wound beneficial agent may be dissolved or dispersed in a solvent prior to applying the coating onto the second side 102b of the adhesive skin contact layer 102. For example, the skin and/or wound beneficial agent may be dissolved in an aqueous solution. The solution may be mixed, and optionally stirred to secure dissolution.

The step of applying the coating on the second side of the adhesive skin contact layer may be achieved with any coating technique.

Preferably, the step of applying the coating on the second side of the adhesive skin contact layer is performed by means of spray coating.

This coating technique is beneficial as it is simple and results in a discontinuous coating onto the adhesive surface. Selected areas of the adhesive layer may be coated and the size of the droplets on the surface may be controlled to avoid interfering with the adhesive properties of the adhesive skin contact layer.

Subsequently, the applied coating is typically dried.

Drying is performed by means well known to the skilled person. For example, the coating may be heated to enhance the evaporation of liquid from the coating.

Preferably, the plurality of protrusions 105 on the first side 103a of the release liner 103 are formed as an in-line step in the process.

Accordingly, the process is significantly simplified, and is also advantageous from a storage perspective. This allows the release liner to be stored on e.g. a roll in a flat condition, requiring much less space than if the release liner would be provided with protrusions.

Suitably, the plurality of protrusions may be formed by embossing.

Embossing is a simple technique to form three dimensional features, e.g. protrusions in a layer. Furthermore, embossing is a suitable technique in embodiments where the protrusions are formed as an in-line step in the process.

For example, the release liner may be subject to an embossing tool on the second side of the release liner, which form protrusions 105 on the first side 103a and corresponding depressions 112 on the second side 105 of the release liner. The embossing tool may be arranged in the process line and embossed protrusions may be provided prior to application of the release liner to the adhesive skin contact layer 102.

The present disclosure is not limited to forming the protrusions by embossing.

In alternative embodiments, the protrusions may be formed by molding or casting.

In the various embodiments described hereinbefore, the top layer may be a film, sheet or membrane that is vapor permeable. Examples of suitable materials for the top layer include, but are not limited to polyurethane, polyethylene or polyamide films, silicone films, polyester based nonwoven materials, and laminates of polyester-based nonwoven materials and polyurethane films. Suitably, the top layer is a polyurethane film. The top layer may have a thickness of from 5 to 40 µm, e.g. from 15 to 25 µm. A thin layer of adhesive, such as a polyacrylate adhesive, may be applied to the top layer 101 to attach the top layer 101 to the adhesive skin contact layer 102 or, where present, an absorbent pad 108 or any other layer of the dressing.

The adhesive skin contact layer preferably comprises a silicone-based adhesive.

Examples of suitable silicone-based adhesives include the two component RTV systems, such as Q72218 (Dow Corning), and SilGel 612 (Wacker Chemie AG) mentioned herein, as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the silicone-based adhesive coating is typically at least 20 µm. The thickness of the silicone-based adhesive coating may be from 40 to 300 µm.

Preferably, the adhesive skin contact layer comprises a plurality of perforations (denoted 113 in figures 2 and 3).

If the adhesive skin contact layer comprises a polymeric film and a silicone-based adhesive coating, the perforations extend through both of these layer.

The adhesive skin contact layer is arranged to receive body fluids, e.g. wound exudate from the wound while the absorbent pad functions to absorb the wound exudate and transport it away from the wound by evaporating it from the top of the dressing; i.e. through the top layer 101.

### Examples

### Example 1: Evaluation of the migration of chlorhexidine gluconate (CHG) to release liner

Tests were performed to evaluate the migration of chlorhexidine gluconate (CHG) from the adhesive skin contact layer to the release liner.

The dressing samples evaluated contained a 20 µm polyurethane film as a the top layer, an adhesive skin contact layer comprising a polymeric film (a 20 µm polyurethane film) coated with a silicone adhesive with varying thicknesses (see below), and a 100 µm polyethylene film as the release liner. The dressings tested differed by the thickness of the silicone adhesive coating, and the characteristics of the release liner, respectively.
Sample A: 50 gsm thick silicone adhesive coating, flat release liner
Sample B: 50 gsm thick silicone adhesive coating, embossed release liner with a pattern of pyramidal shaped protrusions (see figure 5)
Sample C: 200 gsm thick silicone adhesive coating, flat release liner
Sample D: 200 gsm thick silicone adhesive coating, embossed release liner with a pattern of protrusions as illustrated in figures 4a-b.

The height of the protrusions for sample B was about 0.4 mm, whereas the height of the protrusions for sample D was about 1.0 mm. The protrusions of sample D had a substantially flat top surface (with a surface area of about 1.5 mm2 and an angle between the top surface and the sidewalls of about 90 degrees). The protrusions of sample B had a surface area of about 0.3 mm2 and an angle between the top surface and the sidewalls of about 45 degrees.

An aqueous solution of CHG (0.56g/L) was prepared and subsequently substantially uniformly sprayed onto the silicone adhesive layer, to achieve a target concentration of approximately 55 mg/cm2 (40-70 mg/cm2). The samples were dried before three different variants of release liners (see above) were applied onto the silicone surface.

The samples were sent to sterilization on the production site to undergo typical sterilization cycle required for wound dressings (EtO sterilization).

After sterilization and about 8 weeks of storage at ambient temperature, the migration of CHG was evaluated.

To evaluate the migration of CHG, the sterilized samples were first punched into disks of 3.14 cm². The release liners were separated from the adhesive skin contact layer, and placed into separate wells in contact with 1 ml solution of water during a time period of 24 hours to allow full dissolution of the CHG particles. After calibration of the equipment against the solutions of interest, the concentration of CHG in the different wells was evaluated by Liquid chromatography-mass spectrometry to quantify the amount of CHG present on the silicone surface and the amount that migrated to the release liner.

In each Sample category A-D, three dressing samples were tested.

The results are illustrated in table 1 below.

**Table 1: Migration of CHG to the release liner**

| Sample | Average amount of CHG detected on adhesive layer (µg /cm2) | Average amount CHG detected on release liner (µg /cm2) | Total average amount of CHG detected on the adhesive layer and the release liner (µg /cm2) | % detected CHG on release liner |
|---|---|---|---|---|
| A | < LOQ | 63 | 63 | 100 |
| B | 34 | 12 | 45 | 26 |
| C | < LOQ | 67 | 67 | 100 |
| D | 22 | 21 | 43 | 49 |

As illustrated in table 1, the migration of CHG to the release liner was significantly reduced when a protruded release liner was utilized (samples B and D). CHG has an enhanced tendency to migrate to the release liner, so these results are surprising.

### Example 2: Evaluation of the migration of PHMB to release liner

Additional tests were carried out to evaluate the migration of polyhexamethylene biguanide (PHMB) from the release liner to the adhesive skin contact layer.

The test set-up was similar to the set-up explained in Example 1.

The dressing samples evaluated contained a 20 µm polyurethane film as a the top layer, an adhesive skin contact layer comprising a polymeric film (a 20 µm polyurethane film) coated with a 60 gsm silicone adhesive, and a 100 µm polyethylene film as the release liner.

The dressings tested differed by the characteristics of the release liner, respectively.
Sample E: flat release liner
Sample F: release liner with a pattern of pyramidal shaped protrusions (see figure 5).

An aqueous solution of PHMB (7 g/L i.e. 0.7%) was prepared and subsequently substantially uniformly sprayed onto the silicone adhesive layer, with a target concentration of approximately 50 mg/cm2. The samples were dried in an oven (about 2 min, 90°C).

After drying, two variants of release liners (see above) were attached to the spray coated silicone adhesive layer.

The samples were sent to sterilization on the production site to undergo typical sterilization cycle required for wound dressings (EtO sterilization).

After sterilization and about 8 weeks of storage at ambient temperature, the migration of PHMB was evaluated.

To understand the migration of the PHMB, the sterilized samples were punched into disks of 3.8 cm². Triplicates of the adhesive skin contact layers and the release liners of the two dressing variants (E and F) were separated and placed in separate wells in contact with 5 ml solution of water during a time period to allow full dissolution of PHMB (24 hours). The concentration of PHMB in each well was analyzed by UV-vis spectroscopy. The amount of PHMB found on each material surface for sample E and F is presented in table 2 hereinbelow.

**Table 2: Migration of PHMB to the release liner**

| Sample | Average amount of PHMB detected on adhesive layer (µg/cm2) | Average amount PHMB detected on release liner (µg /cm2) | Total average amount of PHMB detected on the adhesive layer and the release liner (µg/cm2) | % detected PHMB on release liner |
|---|---|---|---|---|
| E | 29 | 16 | 45 | 36 |
| F | 50 | 3 | 53 | 6 |

As illustrated in table 2, the migration of PHMB to the release liner was significantly reduced when a protruded release liner was utilized (sample F). PHMB does not migrate as easily as CHG, but the reduced migration to the release liner is a significant improvement even for this substance.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

## Claims

1. A medical dressing (100) comprising a top layer (101), an adhesive skin contact layer (102) and a release liner (103) detachably attached to at least a portion of said adhesive skin contact layer (102); said adhesive skin contact layer (102) having a first side (102a) facing said top layer and a second side (102b) facing said release liner (103), wherein said second side (102b) of said adhesive skin contact layer (102) is provided with a coating (104) comprising a skin and/or wound beneficial agent configured to be released from said adhesive skin contact layer (102) during use, wherein said release liner (103) has a first side (103a) facing said second side (102b) of said adhesive skin contact layer (102), and an opposing, second side (103b), wherein said first side (103a) of said release liner (103) comprises a plurality of protrusions (105), wherein said plurality of protrusions (105) are spaced apart from one another by non-protruding areas (106), wherein said non-protruding areas (106) form gaps (107) between said first side (103a) of said release liner (103) and said second side (102b) of said adhesive skin contact layer (102).

2. The medical dressing (100) according to claim 1, wherein said coating (104) is a discontinuous coating.

3. The medical dressing (100) according to claim 1 or claim 2, wherein said coating (104) is soluble in an aqueous medium.

4. The medical dressing (100) according to claim 1 or claim 2, wherein said skin and/or wound beneficial agent is an antimicrobial agent, preferably an antimicrobial salt.

5. The medical dressing (100) according to any one of the preceding claims, wherein the concentration of said skin and/or wound beneficial agent in said coating is from 1 to 1000 µg/cm2, preferably from 5 to 500 µg/cm2, more preferably from 10 to 200 µg/cm2.

6. The medical dressing (100) according to any one of the preceding claims, wherein said first side (103a) of said release liner (103) is detachably attached to from 5 to 75 %, preferably from 10 to 70%, most preferably from 10 to 50% of the surface area of said second side (102b) of said adhesive skin contact layer (102).

7. The medical dressing (100) according to any one of the preceding claims, wherein the height of each of said protrusions (105) is from 0.1 to 2.0 mm, preferably from 0.2 to 1.5 mm, most preferably from 0.3 to 1.2 mm.

8. The medical dressing (100) according to any one of the preceding claims, wherein said medical dressing (100) comprises an absorbent pad (108) arranged between said top layer (101) and said adhesive skin contact layer (102), and wherein said plurality of protrusions (105) are arranged in an area on the first side (103a) of said release liner (103) that underlies said absorbent pad (108).

9. The medical dressing (100) according to any one of the preceding claims, wherein said adhesive skin contact layer (102) comprises a silicone-based adhesive coating.

10. The medical dressing (100) according to any one of the preceding claims, wherein said adhesive skin contact layer (102) comprises a polymeric film provided with a silicone-based adhesive coating, preferably wherein said silicone-based adhesive coating has a thickness of from 35 to 300 gsm, preferably from 45 to 250 gsm.

11. The medical dressing (100) according to any one of the preceding claims, wherein each of the plurality of protrusions has a substantially flat top surface (109); said top surface (109) forming the contact surface for detachable attachment of said release liner (103) to said adhesive skin contact layer (102).

12. A process for manufacturing a medical dressing (100) comprising:
a) providing a dressing (100) comprising a top layer (101) and an adhesive skin contact layer (102); said adhesive skin contact layer (102) having a first side (102a) facing said top layer (101) and a second, opposing side (102b),
b) applying a coating (104) onto said second side (102b) of said adhesive skin contact layer (102), wherein said coating (104) comprises a skin and/or wound beneficial agent configured to be released from said adhesive skin contact layer (102) during use,
c) providing a release liner (103) having a first side (103a) and an opposing second side (103b), wherein at least a portion of said first side (103a) of said release liner (103) comprises a plurality of protrusions (105);
d) applying said release liner (103) onto said adhesive skin contact layer (102) such that said plurality of protrusions (105) become detachably attached to said second side (102b) of said adhesive skin contact layer (102), wherein said plurality of protrusions (105) are spaced apart from one another by non-protruding areas (106), wherein said non-protruding areas (106) form gaps (107) between said first side (103a) of said release liner (103) and said second side (102b) of said adhesive skin contact layer (102).

13. The process according to claim 12, wherein said step b) of applying a coating (104) is performed by spray coating.

14. The process according to claim 12 or claim 13, wherein said plurality of protrusions (105) are formed as an in-line step in said process, preferably by means of embossing.

## Patentansprüche

1. Medizinischer Verband (100), der eine obere Schicht (101), eine haftende Hautkontaktschicht (102) und eine Trennschicht (103) umfasst, die lösbar an zumindest einem Abschnitt der haftenden Hautkontaktschicht (102) angebracht ist; wobei die haftende Hautkontaktschicht (102) eine erste Seite (102a), die der oberen Schicht zugewandt ist, und eine zweite Seite (102b) aufweist, die der Trennschicht (103) zugewandt ist, wobei die zweite Seite (102b) der haftenden Hautkontaktschicht (102) mit einer Beschichtung (104) versehen ist, die ein Mittel mit positiver Wirkung auf Haut und/oder Wunden umfasst, das so ausgelegt ist, dass es während des Gebrauchs aus der haftenden Hautkontaktschicht (102) freigesetzt wird, wobei die Trennschicht (103) eine erste Seite (103a), die der zweiten Seite (102b) der haftenden Hautkontaktschicht (102) zugewandt ist, und eine gegenüberliegende zweite Seite (103b) aufweist, wobei die erste Seite (103a) der Trennschicht (103) eine Vielzahl von Vorsprüngen (105) umfasst, wobei die Vielzahl von Vorsprüngen (105) voneinander durch nicht vorspringende Bereiche (106) beabstandet ist, wobei die nicht vorspringenden Bereiche (106) Lücken (107) zwischen der ersten Seite (103a) der Trennschicht (103) und der zweiten Seite (102b) der haftenden Hautkontaktschicht (102) bilden.

2. Medizinischer Verband (100) nach Anspruch 1, wobei die Beschichtung (104) eine unterbrochene Beschichtung ist.

3. Medizinischer Verband (100) nach Anspruch 1 oder Anspruch 2, wobei die Beschichtung (104) in einem wässrigen Medium löslich ist.

4. Medizinischer Verband (100) nach Anspruch 1 oder Anspruch 2, wobei das Mittel mit positiver Wirkung auf Haut und/oder Wunden ein antimikrobielles Mittel, vorzugsweise ein antimikrobielles Salz ist.

5. Medizinischer Verband (100) nach einem der vorhergehenden Ansprüche, wobei die Konzentration des Mittels mit positiver Wirkung auf Haut und/oder Wunden in der Beschichtung von 1 bis 1000 µg/cm², vorzugsweise von 5 bis 500 µg/cm², bevorzugter von 10 bis 200 µg/cm² beträgt.

6. Medizinischer Verband (100) nach einem der vorhergehenden Ansprüche, wobei die erste Seite (103a) der Trennschicht (103) lösbar an von 5 bis 75 %, vorzugsweise von 10 bis 70 %, am meisten bevorzugt von 10 bis 50 % der Oberfläche der zweiten Seite (102b) der haftenden Körperkontaktschicht (102) angebracht ist.

7. Medizinischer Verband (100) nach einem der vorhergehenden Ansprüche, wobei die Höhe von jedem der Vorsprünge (105) von 0,1 bis 2,0 mm, vorzugsweise von 0,2 bis 1,5 mm, am meisten bevorzugt von 0,3 bis 1,2 mm beträgt.

8. Medizinischer Verband (100) nach einem der vorhergehenden Ansprüche, wobei der medizinische Verband (100) ein absorbierendes Kissen (108) umfasst, das zwischen der oberen Schicht (101) und der haftenden Hautkontaktschicht (102) angeordnet ist, und wobei die Vielzahl von Vorsprüngen (105) in einem Bereich auf der ersten Seite (103a) der Trennschicht (103) angeordnet ist, der unter dem absorbierenden Kissen (108) liegt.

9. Medizinischer Verband (100) nach einem der vorhergehenden Ansprüche, wobei die haftende Hautkontaktschicht (102) eine Klebstoffbeschichtung auf Silikonbasis umfasst.

10. Medizinischer Verband (100) nach einem der vorhergehenden Ansprüche, wobei die haftende Hautkontaktschicht (102) einen Polymerfilm umfasst, der mit einer Klebstoffbeschichtung auf Silikonbasis versehen ist, wobei vorzugsweise die Klebstoffbeschichtung auf Silikonbasis eine Dicke von 35 bis 300 gsm, vorzugsweise von 45 bis 250 gsm aufweist.

11. Medizinischer Verband (100) nach einem der vorhergehenden Ansprüche, wobei jeder von der Vielzahl von Vorsprüngen eine im Wesentlichen ebene obere Fläche (109) aufweist; wobei die obere Fläche (109) die Kontaktfläche zum lösbaren Anbringen der Trennschicht (103) an der haftenden Hautkontaktschicht (102) bildet.

12. Verfahren zum Herstellen eines medizinischen Verbands (100), umfassend:
a) Bereitstellen eines Verbands (100), der eine obere Schicht (101) und eine haftende Hautkontaktschicht (102) umfasst; wobei die haftende Hautkontaktschicht (102) eine erste Seite (102a), die der oberen Schicht (101) zugewandt ist, und eine zweite gegenüberliegende Seite (102b) aufweist,
b) Aufbringen einer Beschichtung (104) auf die zweite Seite (102b) der haftenden Hautkontaktschicht (102), wobei die Beschichtung (104) ein Mittel mit positiver Wirkung auf Haut und/oder Wunden umfasst, das so ausgelegt ist, dass es während des Gebrauchs aus der haftenden Hautkontaktschicht (102) freigesetzt wird,
c) Bereitstellen einer Trennschicht (103), die eine erste Seite (103a) und eine gegenüberliegende zweite Seite (103b) aufweist, wobei zumindest ein Abschnitt der ersten Seite (103a) der Trennschicht (103) eine Vielzahl von Vorsprüngen (105) umfasst;
d) Aufbringen der Trennschicht (103) derart auf die haftende Hautkontaktschicht (102), dass die Vielzahl von Vorsprüngen (105) lösbar an der zweiten Seite (102b) der haftenden Hautkontaktschicht (102) angebracht ist, wobei die Vielzahl von Vorsprüngen (105) voneinander durch nicht vorspringende Bereiche (106) beabstandet ist, wobei die nicht vorspringenden Bereiche (107) Lücken (107) zwischen der ersten Seite (103a) der Trennschicht (103) und der zweiten Seite (102b) der haftenden Hautkontaktschicht (102) bilden.

13. Verfahren nach Schritt 12, wobei der Schritt b) zum Aufbringen einer Beschichtung (104) mittels Sprühbeschichten erfolgt.

14. Verfahren nach Anspruch 12 oder Anspruch 13, wobei die Vielzahl von Vorsprüngen (105) als integrierter Schritt in dem Verfahren gebildet wird, vorzugsweise mittels Prägen.

## Revendications

1. Pansement médical (100) comprenant une couche supérieure (101), une couche adhésive de contact avec la peau (102) et une doublure détachable (103) fixée de manière détachable à au moins une partie de ladite couche adhésive de contact avec la peau (102) ; ladite couche adhésive de contact avec la peau (102) ayant une première face (102a) faisant face à ladite couche supérieure et une seconde face (102b) faisant face à ladite doublure détachable (103), ladite seconde face (102b) de ladite couche adhésive de contact avec la peau (102) étant pourvue d'un revêtement (104) comprenant un agent bénéfique pour la peau et/ou les plaies configuré pour être détaché de ladite couche adhésive de contact avec la peau (102) pendant l'utilisation, ladite doublure détachable (103) ayant une première face (103a) tournée vers ladite seconde face (102b) de ladite couche adhésive de contact avec la peau (102), et une seconde face opposée (103b), ladite première face (103a) de ladite doublure détachable (103) comprenant une pluralité de saillies (105), ladite pluralité de saillies (105) étant espacées les unes des autres par des zones non saillantes (106), lesdites zones non saillantes (106) formant des espaces (107) entre ladite première face (103a) de ladite doublure détachable (103) et ladite seconde face (102b) de ladite couche adhésive de contact avec la peau (102).

2. Pansement médical (100) selon la revendication 1, dans lequel ledit revêtement (104) est un revêtement discontinu

3. Pansement médical (100) selon la revendication 1 ou la revendication 2, dans lequel ledit revêtement (104) est soluble dans un milieu aqueux.

4. Pansement médical (100) selon la revendication 1 ou la revendication 2, dans lequel ledit agent bénéfique pour la peau et/ou les plaies est un agent antimicrobien, de préférence un sel antimicrobien.

5. Pansement médical (100) selon l'une quelconque des revendications précédentes, dans lequel la concentration dudit agent bénéfique pour la peau et/ou les plaies dans ledit revêtement est de 1 à 1000 µg/cm2, de préférence de 5 à 500 µg/cm2, plus préférablement de 10 à 200 µg/cm2.

6. Pansement médical (100) selon l'une quelconque des revendications précédentes, dans lequel ladite première face (103a) de ladite doublure détachable (103) est fixée de manière détachable à 5 à 75 %, de préférence à 10 à 70 %, de manière tout à fait préférée à 10 à 50 % de la superficie de ladite seconde face (102b) de ladite couche adhésive de contact avec la peau (102).

7. Pansement médical (100) selon l'une quelconque des revendications précédentes, dans lequel la hauteur de chacune desdites saillies (105) est de 0,1 à 2,0 mm, de préférence de 0,2 à 1,5 mm, mieux encore de 0,3 à 1,2 mm.

8. Pansement médical (100) selon l'une quelconque des revendications précédentes, dans lequel ledit pansement médical (100) comprend un tampon absorbant (108) agencé entre ladite couche supérieure (101) et ladite couche adhésive de contact avec la peau (102), et dans lequel ladite pluralité de saillies (105) sont agencées dans une zone sur la première face (103a) de ladite doublure détachable (103) qui est sous ledit tampon absorbant (108).

9. Pansement médical (100) selon l'une quelconque des revendications précédentes, dans lequel ladite couche adhésive de contact avec la peau (102) comprend un revêtement adhésif à base de silicone.

10. Pansement médical (100) selon l'une quelconque des revendications précédentes, dans lequel ladite couche adhésive de contact avec la peau (102) comprend un film polymère muni d'un revêtement adhésif à base de silicone, ledit revêtement adhésif à base de silicone ayant de préférence une épaisseur de 35 à 300 gsm, de préférence de 45 à 250 gsm.

11. Pansement médical (100) selon l'une quelconque des revendications précédentes, dans lequel chacune de la pluralité de saillies a une surface supérieure sensiblement plate (109) ; ladite surface supérieure (109) formant la surface de contact pour la fixation détachable de ladite doublure détachable (103) à ladite couche adhésive de contact avec la peau (102).

12. Procédé de fabrication d'un pansement médical (100) comprenant :
a) la prévision d'un pansement (100) comprenant une couche supérieure (101) et une couche adhésive de contact avec la peau (102) ; ladite couche adhésive de contact avec la peau (102) ayant une première face (102a) faisant face à ladite couche supérieure (101) et une seconde face opposée (102b),
b) l'application d'un revêtement (104) sur ladite seconde face (102b) de ladite couche adhésive de contact avec la peau (102), ledit revêtement (104) comprenant un agent bénéfique pour la peau et/ou les plaies configuré pour être détaché de ladite couche adhésive de contact avec la peau (102) pendant l'utilisation,
c) la prévision d'une doublure détachable (103) ayant une première face (103a) et une seconde face opposée (103b), au moins une partie de ladite première face (103a) de ladite doublure détachable (103) comprenant une pluralité de saillies (105) ;
d) l'application de ladite doublure détachable (103) sur ladite couche adhésive de contact avec la peau (102) de telle sorte que ladite pluralité de saillies (105) se fixent de manière détachable à ladite seconde face (102b) de ladite couche adhésive de contact avec la peau (102), ladite pluralité de saillies (105) étant espacées les unes des autres par des zones non saillantes (106), lesdites zones non saillantes (107) formant des espaces (107) entre ladite première face (103a) de ladite doublure détachable (103) et ladite seconde face (102b) de ladite couche adhésive de contact avec la peau (102).

13. Procédé selon la revendication 12, dans lequel ladite étape b) d'application d'un revêtement (104) est effectuée par revêtement par pulvérisation.

14. Procédé selon la revendication 12 ou la revendication 13, dans lequel ladite pluralité de saillies (105) sont formées comme une étape en ligne dans ledit procédé, de préférence au moyen d'un gaufrage.
